(19) Europäisches Patentamt
European Patent Office
Office européen des brevets

(11) Numéro de publication : **0 097 585**
**B1**

(12) # FASCICULE DE BREVET EUROPÉEN

(45) Date de publication du fascicule du brevet : **26.03.86**

(51) Int. Cl.[4] : **C 07 D215/22**

(21) Numéro de dépôt : **83401242.9**

(22) Date de dépôt : **16.06.83**

(54) **Procédé de préparation d'hydroxy-4 quinoléines.**

(30) Priorité : **17.06.82 FR 8210615**

(43) Date de publication de la demande : **04.01.84 Bulletin 84/01**

(45) Mention de la délivrance du brevet : **26.03.86 Bulletin 86/13**

(84) Etats contractants désignés : **AT BE CH DE FR GB IT LI LU NL SE**

(56) Documents cités :
**EP-A- 0 056 765**
**US-A- 2 558 211**
**Le dossier contient des informations techniques présentées postérieurement au dépôt de la demande et ne figurant pas dans le présent fascicule.**

(73) Titulaire : **RHONE-POULENC SANTE**
**Les Miroirs 18 Avenue d'Alsace**
**F-92400 Courbevoie Cedex (FR)**

(72) Inventeur : **Michelet, Daniel**
**24 Chemin de Montribloud**
**F-69160 Tassin (FR)**
Inventeur : **Petre, Dominique**
**8 rue Fantin-Latour**
**F-75016 Paris (FR)**

(74) Mandataire : **Pilard, Jacques et al**
**RHONE-POULENC RECHERCHES Service Brevets Pharma 25, Quai Paul Doumer**
**F-92408 Courbevoie Cedex (FR)**

**EP 0 097 585 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un procédé de préparation d'hydroxy-4 quinoléines de formule générale :

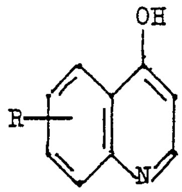

(I)

dans laquelle R représente un atome d'hydrogène ou 1 à 3 substituants identiques ou différents choisis parmi les atomes d'halogène, les radicaux alcoyles contenant 1 à 4 atomes de carbone, alcoyloxyles dont la partie alcoyle contient 1 à 4 atomes de carbone ou trifluorométhyle en position 2, 3, 5, 6, 7 ou 8 à partir des tétrahydro-1,2,3,4 quinolinones-4 correspondantes de formule générale :

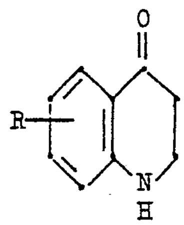

(II)

dans laquelle R est défini comme précédemment.

Les produits de formule générale (I) sont des intermédiaires particulièrement intéressants pour la synthèse de produits thérapeutiquement actifs, tels que la glafénine [ester dihydroxypropylique de l'acide N-(chloro-7 quinoyl-4) anthranilique] ou la floctafénine [ester dihydroxypropylique de l'acide N-(trifluorométhyl-8 quinolyl-4) anthranilique qui sont des analgésiques puissants, la chloroquine [chloro-7 (diéthylamino-4 méthyl-1 butylamino)-4 quinoléine] ou l'amodiaquine [chloro-7 (diéthylaminométhyl-3 hydroxy-4 phénylamino)-4 quinoléine] qui présentent des propriétés antimalariques remarquables.

Il est connu de préparer une hydroxy-4 quinoléine à partir de la quinolinone-4 correspondante soit par déshydrogénation catalytique en présence de palladium sur charbon [W. S. JOHNSON et B. G. BUELL, J. Amer. Chem. Soc., *74*, 4513 (1952)], soit par transfert d'hydrogène catalysé par le palladium sur charbon en présence d'acide maléique (brevet des Etats-Unis d'Amérique 2 558 211). Cependant, lorsque l'on utilise une tétrahydro-1,2,3,4 quinolinone-4 de formule générale (II) dans laquelle R représente un atome d'halogène, l'aromatisation s'accompagne d'une déhalogénation ce qui conduit à un mélange d'hydroxy-4 quinoléine halogénée et d'hydroxy-4 quinoléine.

Il a maintenant été trouvé, et c'est ce qui fait l'objet de la présente invention, que l'hydroxy-4 quinoléine de formule générale (I) peut être obtenue par oxydation en milieu basique de la tétrahydro-1,2,3,4 quinolinone-4 correspondante, sous pression, au moyen d'oxygène ou d'air en excès à une température comprise entre 80 et 150 °C.

L'oxydation est réalisée en milieu basique constitué, de préférence, par une solution aqueuse d'un hydroxyde alcalin tel que la soude. Il est particulièrement avantageux d'utiliser de 0,5 à 3 moles d'hydroxyde alcalin par mole de tétrahydro-1,2,3,4 quinolinone-4 mise en œuvre. Généralement, une augmentation de la concentration en hydroxyde alcalin se traduit par une augmentation de la vitesse de réaction.

Pour que la réaction puisse être réalisée, il est nécessaire d'opérer sous une pression généralement comprise entre 2 et 15 bars, l'air ou l'oxygène étant injecté avec un débit convenable à travers le mélange réactionnel agité. Généralement, une augmentation de pression se traduit par un accroissement de la vitesse de réaction.

Pour des raisons de commodité, il est particulièrement intéressant d'opérer à une température comprise entre 90 et 100 °C, sous une pression comprise entre 5 et 10 bars et en utilisant de 1 à 2 moles de soude par mole de tétrahydro-1,2,3,4 quinolinone-4 mise en œuvre. Dans ces conditions la réaction est complète après 2 à 6 heures de chauffage.

La mise en œuvre du procédé selon l'invention sur une halogéno tétrahydro-1,2,3,4 quinolinone-4 permet d'obtenir l'halogéno-hydroxy-4 quinoléine correspondante pratiquement exempte d'hydroxy-4 quinoléine.

L'hydroxy-4 quinoléine de formule générale (I) obtenue selon le procédé de la présente invention peut être séparée du mélange réactionnel et purifiée par application des méthodes habituelles telles que la cristallisation ou la chromatographie.

La tétrahydro-1,2,3,4 quinolinone-4 de formule générale (II) utilisée comme produit de départ peut avantageusement être préparée par cyclisation de l'acide anilino-3 propionique correspondant au moyen d'un mélange d'acide fluorhydrique et de trifluorure de bore.

L'acide anilino-3 propionique peut être obtenu par action d'un excès d'une aniline convenablement substituée sur l'acide acrylique. La réaction est effectuée généralement dans l'eau à une température comprise entre 70 et 100 °C. La durée de la réaction est comprise entre 1 et 4 heures.

La chloro-7 hydroxy-4 quinoléine qui peut être obtenue selon le procédé de la présente invention peut être transformée en glafénine selon le procédé décrit dans le brevet français 2413 M après transformation en dichloro-4,7 quinoléine au moyen, par exemple, d'oxychlorure de phosphore.

Les exemples suivants, donnés à titre non limitatif, montrent comment l'invention peut être mise en pratique.

Exemple 1

Dans un autoclave en acier de 400 $cm^3$, muni d'un agitateur à pales, d'un réfrigérant, d'une entrée et sortie de gaz, on charge :

| | |
|---|---|
| — chloro-7 tétrahydro-1,2,3,4 quinolinone-4 | 18,15 g (100 mM) |
| — soude | 8,0 g (200 mM) |
| — eau | 200 g |

On chauffe le réacteur à 90 °C. La pression est fixée à 10 bars. L'air à 21 % d'oxygène est injecté en excès dans la masse réactionnelle agitée avec un débit massique de 12 g/heure.

Après 2 heures 30 de chauffage, le réacteur est refroidi à une température voisine de 20 °C et la pression est ramenée à la pression atmosphérique.

La masse réactionnelle est extraite avec du chlorure de méthylène. La phase chlorométhylénique est évaporée à sec. On recueille 0,96 g d'un solide jaune clair contenant 98,2 % de chloro-7 tétrahydro-1,2,3,4 quinolinone-4 (5,2 mM) par dosage en chromatographie en phase liquide.

La phase aqueuse est ajustée à pH 6,0 par addition d'acide sulfurique N. Le précipité blanc laiteux est filtré et séché. On recueille 15,92 g d'un produit blanc crème qui contient 98,15 % (en poids) d'hydroxy-4 chloro-7 quinoléine par dosage en chromatographie en phase liquide soit 87,0 mM.

Le filtrat est acidifié à pH 2,5 par addition d'acide sulfurique N, puis extrait avec du chlorure de méthylène. La phase chlorométhylénique est évaporée à sec. On recueille 1,15 g d'un produit brun. Par dosage en chromatographie en phase liquide ce produit contient 0,59 g (3,4 mM) d'hydroxy-4 chloro-7 quinoléine et 0,31 g (1,8 mM) d'acide amino-2 chloro-4 benzoïque.

Sur la phase aqueuse restante on dose, par argentimétrie 2,9 mM d'ions chlorures.

La chloro-7 hydroxy-4 quinoléine est obtenue avec :

— un taux de transformation de 94,8 % de la chloro-7 tétrahydro-1,2,3,4 quinolinone-4

— un rendement en hydroxy-4 chloro-7 quinoléine de 95,3 % par rapport à la chloro-7 tétrahydro-1,2,3,4 quinolinone-4 transformée.

La chloro-7 tétrahydro-1,2,3,4 quinolinone-4 peut être préparée de la manière suivante :

Dans un réacteur en acier inoxydable contenant 50 g d'acide fluorhydrique liquide refroidi à 5 °C on ajoute 10 g d'acide m.chloroanilino-3 propionique (titrant 94,5 %). La solution est saturée de trifluorure de bore gazeux. A cet effet le contenu du réacteur est maintenu à 20 °C puis saturé par le trifluorure de bore gazeux sous une pression de 12 bars pendant 1 heure. Le réacteur est ensuite fermé puis chauffé à 80 °C pendant 20 heures.

Au cours du chauffage la pression s'élève d'abord jusqu'à 20 bars puis diminue progressivement pour se stabiliser vers 16 bars. Le réacteur est ensuite refroidi à 10 °C puis ouvert de manière à laisser échapper le trifluorure de bore. Le liquide rougeâtre obtenu est versé dans un mélange d'eau et de glace. Après extraction 3 fois par 100 $cm^3$ de chloroforme, la couche organique est lavée plusieurs fois par 100 $cm^3$ d'eau jusqu'à un pH compris entre 3 et 4 puis séchée sur sulfate de sodium. Après filtration et concentration à sec sous pression réduite (10 mm de mercure ; 1,33 kPa), on obtient 9 g de chloro-7 tétrahydro-1,2,3,4 quinolinone-4 cristallisée dont le titre déterminé par chromatographie en phase gazeuse est de 94,5 %.

Le taux de transformation est de 100 % et le rendement par rapport à l'acide m.chloroanilino-3 propionique est de 99 %.

Par examen en chromatographie en phase gazeuse, on détermine que la teneur en isomère chloro-5 est voisine de 0,7 %.

Exemples 2 à 9

On opère comme dans l'exemple 1 en partant à chaque fois de 18,15 g (100 mM) de chloro-7 tétrahydro-1,2,3,4 quinolinone-4 en faisant varier les paramètres de la réaction. Les résultats sont rassemblés dans le tableau I dans lequel :

Q = chloro-7 tétrahydro-1,2,3,4 quinolinone-4
OCQ = hydroxy-4 chloro-7 quinoléine
OH/Q = rapport molaire entre la soude et la chloro-7 tétrahydro-1,2,3,4 quinolinone-4
TT = taux de transformation de la chloro-7 tétrahydro-1,2,3,4 quinolinone-4
RT = rendement par rapport à la chloro-7 tétrahydro-1,2,3,4 quinolinone-4 transformée.

| Exemple | Température ( °C) | Pression (bars) | OH/Q | Durée (heures) | Extrait basique (Q) | Précipité pH 6,0 (OCQ) | Extrait pH 2,5 (OCQ) | TT % | RT % |
|---|---|---|---|---|---|---|---|---|---|
| 2 | 83 | 10 | 2 | 4 h 1/4 | 5,4 g (29,4 mM) | 11,7 g (61,9 mM) | 1,1 g (4,2 mM) | 70,5 | 94 |
| 3 | 110 | 10 | 2 | 2 h | 0,4 g (1,6 mM) | 16,6 g (90 mM) | 1,1 g (3,4 mM) | 98,4 | 95,2 |
| 4 | 140 | 10 | 2 | 3 h 20 | 0,2 g (1,1 mM) | 14,3 g (73 mM) | 3,8 g (10 mM) | 98,9 | 84,2 |
| 5 | 90 | 2 | 2 | 6 h 20 | 7,4 g (40 mM) | 10,5 g (57,4 mM) | 1,2 g (2,6 mM) | 60 | 100 |
| 6 | 90 | 5 | 2 | 4 h 1/2 | 1,3 g (6,4 mM) | 15,8 g (81,4 mM) | 1,0 g (3,2 mM) | 93,6 | 90,4 |
| 7 | 90 | 10 | 1,1 | 5 h 1/2 | 4,6 g (24,5 mM) | 12,2 g (65,2 mM) | 1,3 g (3,8 mM) | 75,5 | 91,4 |
| 8 | 90 | 10 | 0,5 | 2 h 1/2 | 11,2 g (62 mM) | 6,2 g (34,1 mM) | 1,0 g (3,2 mM) | 38 | 98,1 |
| 9 | 90 | 10 | 0 | 5 h | 18,1 g (100 mM) | - | - | 0 | 0 |

Exemple 10

Dans un autoclave de 150 $cm^3$ (volume utile 80 $cm^3$), muni d'un agitateur du type « turbine Rushton » (vitesse de rotation 750 tours/minute), d'un réfrigérant et d'une entrée et sortie de gaz, ou charge :

| | |
|---|---|
| — trifluorométhyl-8 tétrahydro-1,2,3,4 quinolinone-4 : | 2,705 g (12,58 mM) |
| — soude | 1 g (25 mM) |
| — eau | 2,5 $cm^3$ |

On chauffe le réacteur à 90 °C. La pression est fixée à 10 bars. De l'air à 21 % d'oxygène est injecté en excès dans la masse réactionnelle agitée avec un débit de 5 litres/heure environ.

Après 7 heures de chauffage, le réacteur est refroidi à une température d'environ 20 °C et la pression est ramenée à la pression atmosphérique.

La masse réactionnelle est extraite avec du chlorure de méthylène. Par évaporation à sec de la phase chlorométhylénique, on obtient 1,913 g (8,9 mM) de produit de départ non transformé contenant des traces de trifluorométhyl-8, hydroxy-4 quinoléine.

La phase aqueuse est ajustée à pH 6,0 par addition d'acide sulfurique N puis est extraite au butanol normal. Le butanol est évaporé sous pression réduite. On recueille ainsi 0,616 g (2,90 mM) de trifluorométhyl-8 hydroxy-4 quinoléine pratiquement pure.

Le taux de transformation est de 29,3 % et le rendement par rapport au produit de départ transformé est de 78,5 %.

La trifluorométhyl-8 tétrahydro-1,2,3,4 quinoléine-4 peut être préparée de la manière suivante :

Dans un réacteur en acier inoxydable contenant 100 g d'acide fluorhydrique liquide refroidi à 5 °C, on ajoute 23,3 g d'acide o-trifluorométhylanilino-3 propionique. La solution est saturée de trifluorure de bore gazeux. A cet effet, le contenu du réacteur est maintenu à 20 °C puis saturé par le trifluorure de bore gazeux sous une pression de 17 bars pendant 1 heure. Le réacteur est ensuite fermé puis chauffé à 80 °C pendant 19 heures.

Après refroidissement à 10 °C, le réacteur est ouvert de manière à laisser échapper le trifluorure de bore. Le liquide obtenu est versé dans un mélange d'eau et de glace. Après extraction par le chloroforme, la couche organique est lavée à l'eau puis séchée sur sulfate de sodium. Après filtration et concentration sous pression réduite (10 mm de mercure ; 1,33 kPa), on obtient 13,9 g de trifluorométhyl-8 tétrahydro-1,2,3,4 quinolinone-4 dont le titre, déterminé par chromatographie en phase gazeuse est de 99 %.

Le taux de transformation est de 68,7 % et le rendement par rapport à l'acide o-trifluorométhyl-anilino-3 propionique transformé est voisin de 95 %.

Exemple 11

On opère comme dans l'exemple 10, en partant de :

| | |
|---|---|
| — méthyl-7 tétrahydro-1,2,3,4 quinolinone-4 | 1,636 g (10,16 mM) |
| — soude | 1 g (25 mM) |
| — eau | 2,5 $cm^3$ |

Le mélange réactionnel est chauffé pendant 14,5 heures dans les conditions de l'exemple 10 puis il est traité de la même manière.

On recueille 0,215 g de produit de départ, ce qui correspond à un taux de transformation de 86,9 % et 1,278 g (8,04 mM) de méthyl-7 hydroxy-4 quinoléine pratiquement pure.

Le rendement par rapport au produit de départ transformé est de 91 %.

La méthyl-7 tétrahydro-1,2,3,4 quinolinone-4 peut être préparée de la manière suivante :

Dans un réacteur en acier inoxydable contenant 100 g d'acide fluorhydrique liquide refroidi à 5 °C, on ajoute 18,5 g d'acide m.méthylanilino-3 propionique. La solution est saturée de trifluorure de bore gazeux. A cet effet, le contenu du réacteur est maintenu à 20 °C puis saturé de trifluorure de bore gazeux sous une pression de 11 bars pendant 1 heure. Le réacteur est ensuite chauffé à 82 °C pendant 23 heures.

Après refroidissement à 10 °C, le réacteur est ouvert de manière à laisser échapper le trifluorure de bore. Le liquide obtenu est versé dans un mélange d'eau et de glace. Après extraction par le chloroforme, la couche organique est lavée à l'eau puis séchée sur sulfate de sodium. Après filtration et concentration à sec sous pression réduite (10 mm de mercure ; 1,33 kPa), on obtient 16,6 g de méthyl-7 tétrahydro-1,2,3,4 quinolinone-4, dont le titre, déterminé par chromatographie en phase gazeuse, est de 96 %.

Le taux de transformation est voisin de 100 % et le rendement par rapport à l'acide m.anilino-3 propionique transformé est voisin de 100 %.

Par examen en chromatographie en phase gazeuse et en résonance magnétique nucléaire, la teneur en isomère méthyl-5 est inférieure à 1 %.

Exemple 12

On opère comme dans l'exemple 10, en partant de :

| | |
|---|---|
| — chloro-7 méthyl-2 tétrahydro-1,2,3,4 quinolinone-4 | 1,937 g (9,9 mM) |
| — soude | 1 g (25 mM) |
| — eau | 2,5 $cm^3$ |

Le mélange réactionnel est chauffé pendant 7,5 heures dans les conditions de l'exemple 10 puis il est traité de la même manière.

On recueille 1,209 g de produit de départ, ce qui représente un taux de transformation de 37,6 % et 0,692 g (3,57 mM) de chloro-7 méthyl-2 hydroxy-4 quinoléine pratiquement pure.

Le rendement par rapport au produit de départ transformé est de 96 %.

La chloro-7 méthyl-2 tétrahydro-1,2,3,4 quinolinone-4 peut être préparée de la manière suivante :

Dans un réacteur en acier inoxydable contenant 100 g d'acide fluorhydrique liquide refroidi à 5 °C, on ajoute 21,4 g d'acide m.chloroanilino-3 butanoïque. La solution est saturée de trifluorure de bore gazeux. A cet effet, le contenu du réacteur est maintenu à 20 °C puis saturé de trifluorure de bore gazeux sous une pression de 10 bars pendant 1 heure. Ce réacteur est ensuite fermé puis chauffé à 82 °C pendant 24 heures.

Après refroidissement à 10 °C, le réacteur est ouvert de manière à laisser échapper le trifluorure de bore. Le liquide obtenu est versé dans un mélange d'eau et de glace. Après extraction par le chloroforme, la couche organique est lavée à l'eau puis séchée sur sulfate de sodium. Après filtration et concentration à sec sous pression réduite (10 mm de mercure ; 1,33 kPa), on obtient 18,3 g de chloro-7 méthyl-2 tétrahydro-1,2,3,4 quinolinone-4 dont le titre, déterminé par chromatographie en phase gazeuse, est de 95 %.

Le taux de transformation est de 93,5 % et le rendement par rapport à l'acide m.chloroanilino-3 butanoïque transformé est voisin de 100 %.

Par examen en chromatographie en phase gazeuse et en résonance magnétique nucléaire, la teneur en isomère chloro-5 est inférieure à 1 %.

**Revendications**

1. Procédé de préparation d'une hydroxy-4 quinoléine de formule générale :

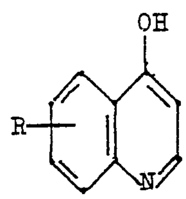

dans laquelle R représente un atome d'hydrogène ou 1 à 3 substituants identiques ou différents choisis parmi les atomes d'halogène, les radicaux alcoyles contenant 1 à 4 atomes de carbone, alcoyloxyles dont la partie alcoyle contient 1 à 4 atomes de carbone ou trifluorométhyle en position 2, 3, 5, 6, 7 ou 8 caractérisé en ce que l'on oxyde une tétrahydro-1,2,3,4 quinolinone-4 de formule générale :

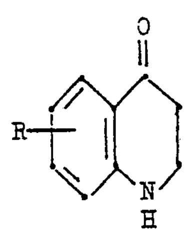

dans laquelle R est défini comme précédemment, en milieu basique, sous pression, au moyen d'un excès d'oxygène ou d'air à une température comprise entre 80 et 150 °C.

2. Procédé selon la revendication 1 caractérisé en ce que le milieu basique est constitué d'une solution aqueuse d'un hydroxyde alcalin.

3. Procédé selon la revendication 2 caractérisé en ce que l'hydroxyde alcalin est la soude.

4. Procédé selon la revendication 2 caractérisé en ce que l'on utilise 0,5 à 3 moles d'hydroxyde alcalin par mole de tétrahydro-1,2,3,4 quinolinone-4 mise en œuvre.

5. Procédé selon la revendication 1 caractérisé en ce que l'on opère sous une pression de 2 à 15 bars.

## Claims

1. Process for the preparation of a 4-hydroxy-quinoline of the general formula :

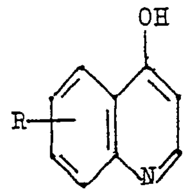

in which R represents a hydrogen atom or 1 to 3 identical or different substituents chosen from among halogen atoms, alkyl radicals containing 1 to 4 carbon atoms, alkoxy radicals of which the alkyl part contains 1 to 4 carbon atoms, or trifluoromethyl, in the 2-, 3-, 5-, 6-, 7- or 8-position, characterised in that a 1,2,3,4-tetrahydroquinolin-4-one of the general formula :

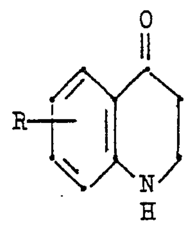

in which R is defined as above is oxidised in a basic medium under pressure by means of an excess of oxygen or air, at a temperature of between 80 and 150 °C.

2. Process according to Claim 1, characterised in that the basic medium consists of an aqueous solution of an alkali metal hydroxide.

3. Process according to Claim 2, characterised in that the alkali metal hydroxide is sodium hydroxide.

4. Process according to Claim 2, characterised in that 0.5 to 3 moles of alkali metal hydroxide are used per mole of 1,2,3,4-tetrahydro-quinolin-4-one employed.

5. Process according to Claim 1, characterised in that it is carried out under a pressure of 2 to 15 bars.

## Patentansprüche

1. Verfahren zur Herstellung eines 4-Hydroxychinolins der allgemeinen Formel :

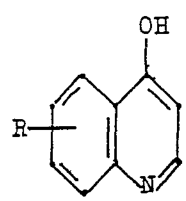

in welcher R ein Wasserstoffatom oder 1 bis 3 gleiche oder verschiedene, unter den Halogenatomen, den Alkylresten mit 1 bis 4 Kohlenstoffatomen, Alkyloxylresten, deren Alkylteil 1 bis 4 Kohlenstoffatome enthält, oder Trifluormethylresten in 2-, 3-, 5-, 6-, 7- oder 8-Stellung ausgewählte Substituenten bedeutet, dadurch gekennzeichnet, daß man ein 1,2,3,4-Tetrahydrochinolin-4-on der allgemeinen Formel :

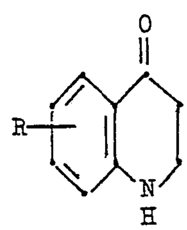

in welcher R wie oben definiert ist, in basischem Milieu unter Druck mittels Sauerstoff oder Luft im Überschuß bei einer Temperatur zwischen 80 °C und 150 °C oxidiert.

2. Verfahren nach Anspruch 1, dadurch gekennzeichnet, daß das basische Milieu aus einer wässerigen Lösung eines alkalihydroxids besteht.

3. Verfahren nach Anspruch 2, dadurch gekennzeichnet, daß das Alkalihydroxid das Natriumhydroxid ist.

4. Verfahren nach Anspruch 2, dadurch gekennzeichnet, daß man 0,5 bis 3 Mole Alkalihydroxid pro eingesetztem Mol 1,2,3,4-Tetrahydrochinolin-4-on verwendet.

5. Verfahren nach Anspruch 1, dadurch gekennzeichnet, daß man unter einem Druck von 2 bis 15 bar arbeitet.